# EUROPEAN PATENT APPLICATION

(11) **EP 3 692 897 A1**
(43) Date of publication of application: **12.08.2020**
(21) Application number: 18929614.8
(22) Date of filing: 20.12.2018
(51) Int. Cl.: A61B 5/01

(54) **GUIDED ENERGY RELEASE METHOD AND DEVICE BASED ON THREE-DIMENSIONAL SPATIAL SKIN TEMPERATURE TOPOGRAPHIC MAP**

(30) Priority: 06.08.2018 CN 201810885773
(71) Applicant: OHMK (Tianjin) Medical Technology Co., Ltd., Tianjin 301700 (CN)
(72) Inventor: YUAN, Yibing, Tianjin 301700 (CN)
(74) Representative: Lusinchi, Laurent Pierre
(86) International application number: PCT/CN2018/122452
(87) International publication number: WO 2020/029511

(57) **Abstract**

The present invention discloses a method for guiding and releasing energy based on a three-dimensional skin temperature topographic map, and also relates to a corresponding device for guiding and releasing energy. The device for guiding and releasing energy performs dynamic closed-loop control on a temperature sensor and an interventional energy release unit, and eventually controls a temperature value of skin tissue to be within an optimal temperature range to achieve dynamic balance between interventional energy therapy and dynamic monitoring and diagnosis. The three-dimensional skin temperature topographic map is displayed according to spatial location data and real-time temperature data, so that an operator is provided with intuitive and clear operation instructions.

## Description

### BACKGROUND

### Technical Field

The present invention relates to a method for guiding and releasing energy to skin tissue, and in particular, to a method for guiding and releasing energy based on a three-dimensional skin temperature topographic map, also relates to a corresponding device for guiding and releasing energy, and belongs to the technical field of interventional energy therapy.

### Related Art

Interventional energy therapy refers to directionally releasing energy to a specific lesion location by using a non-contact energy releasing technology of releasing energy such as radio frequency energy, ultrasonic energy, laser energy or microwave energy, so that the lesion location is stimulated to generate an expected response, to achieve a planned disease treatment effect (or medical cosmetic effect). The currently popular laser rejuvenation technology is a typical measure of interventional energy therapy.

The temperature of skin tissue is affected by subcutaneous blood circulation, metabolism of local tissue, thermal conductivity of skin, and temperature and humidity exchange between skin and environment. Changes in the temperature of skin tissue may be directly affected by local blood, thermal changes of tissue, thermal conduction in tissue, nerve reflexes, physical stress, environmental temperature, humidity, air pressure, thermal transfer conditions, and wind speed, and the like. The temperature of skin tissue reflects a health condition of subcutaneous tissue or even deep tissue, and deep lesions in skin tissue may be diagnosed through thermal imaging. In contrast, interventional energy therapy is performed by positioning skin tissue, so that a treatment effect may be produced on lesions in subcutaneous tissue. For example, energy is released by positioning skin tissue, so that subcutaneous blood circulation is accelerated, and the metabolism of local tissue is enhanced. Energy is released to the dermis and the epidermis is kept at a normal temperature, so that the dermis of skin may become thicker and wrinkles may become shallow or disappear. Subcutaneous collagen is shrunk, tightened, and reshaped, and new collagen is generated, so that anti-aging and cosmetic effects are achieved.

Generally, existing interventional energy therapy is still in an open-loop stage. That is, energy intervention to a lesion location is a step-by-step practice. The treatment effect of the interventional energy then needs to be diagnosed, and energy intervention is performed another time according to a diagnosis result. The entire process requires the supervision of a doctor. In addition, a specific amount of energy to be released needs to be determined by a doctor according to experience, and sometimes, it is difficult to determine an accurate amount.

### SUMMARY

A main technical problem to be resolved by the present invention is to provide a method for generating a three-dimensional skin temperature topographic map.

Another technical problem to be resolved by the present invention is to provide a method for guiding and releasing energy based on a three-dimensional skin temperature topographic map.

Still another technical problem to be resolved by the present invention is to provide a device for guiding and releasing energy based on a three-dimensional skin temperature topographic map.

To achieve the foregoing objectives of the present invention, the following technical solutions are used in the present invention:
According to a first aspect of the present invention, a method for generating a three-dimensional skin temperature topographic map is provided, where the method is implemented based on an inertial navigation unit and a temperature sensor, where relative locations of the inertial navigation unit and the temperature sensor are kept fixed, and the method includes the following steps:
recording and drawing, by the inertial navigation unit, a three-dimensional curve along which the temperature sensor moves on a skin surface, to obtain spatial location data, where the temperature sensor records real-time temperature data at each spatial location simultaneously; and
integrating the spatial location data and corresponding real-time temperature data to obtain a three-dimensional skin temperature topographic map.

Preferably, the inertial navigation unit obtains a location and a speed by performing integration on the acceleration of an object measured by an accelerometer; and obtains an angle of an object by integrating an angular increment of an object measured by a gyroscope, and corrects the acceleration of the object in an inertial system by using angle information.

According to a second aspect of the present invention, a method for guiding and releasing energy based on a three-dimensional skin temperature topographic map is provided, including the following steps:
performing interventional energy therapy on skin tissue, and measuring a dynamic response temperature of the skin tissue by using a temperature sensor; and
feeding back the dynamic response temperature of the skin tissue through closed-loop control, to dynamically adjust a release speed and a release direction of interventional energy.

Preferably, the method further includes the following steps:
generating a real-time three-dimensional skin temperature topographic map by combining the inertial navigation unit and the temperature sensor; and
displaying the three-dimensional skin temperature topographic map by using a display apparatus, so that an operator knows an external effect of the interventional energy therapy in real time and adjusts the release speed and release direction of interventional energy in a targeted manner.

According to a third aspect of the present invention, a device for guiding and releasing energy based on a three-dimensional skin temperature topographic map is provided, including a temperature sensor, a data collection apparatus, a microprocessor, an inertial navigation unit, and an interventional energy release unit, where
the temperature sensor, the interventional energy release unit, and the inertial navigation unit are fixed together and are tightly attached to skin tissue during use;
real-time temperature data collected by the temperature sensor is input into the data collection apparatus to enter the microprocessor; and
the microprocessor dynamically adjusts and controls interventional energy of the interventional energy release unit according to the real-time temperature data collected by the temperature sensor.

Preferably, spatial location data collected by the inertial navigation unit also enters the microprocessor; and the microprocessor integrates the spatial location data and the corresponding real-time temperature data to obtain a three-dimensional skin temperature topographic map and displays the three-dimensional skin temperature topographic map in real time by using a display apparatus.

Preferably, the temperature sensor collects in real time a temperature value of the skin tissue at which the temperature sensor is located, compares this accurate temperature value with a temperature value within an optimal temperature range that is clinically verified, and sends a comparison result into the microprocessor; and a closed-loop control algorithm is performed in the microprocessor to control a value and a frequency of energy released by the interventional energy release unit, to control the temperature value of the skin tissue to be within the optimal temperature range.

Preferably, the device for guiding and releasing energy is a beauty instrument implementing a skin care function.

Preferably, the interventional energy release unit is any one of an ultrasonic generator, a radio frequency transmitter, or a pulse laser.

Preferably, the temperature sensor is a thermal couple, a temperature sensitive diode, a platinum thin-film thermistor, or any one of an infrared temperature measurement sensor, an infrared array temperature measurement sensor, or an imaging charge-coupled device (CCD) temperature measurement sensor.

Compared with the prior art, in the present invention, dynamic closed-loop control is performed on the temperature sensor and the interventional energy release unit, and a temperature value of skin tissue is eventually controlled to be within an optimal temperature range to achieve dynamic balance between interventional energy therapy and dynamic monitoring and diagnosis. In addition, by means of the present invention, the three-dimensional skin temperature topographic map is displayed according to the spatial location data and the real-time temperature data, so that an operator is provided with intuitive and clear operation instructions.

### BRIEF DESCRIPTION OF THE DRAWINGS

FIG. 1 is a schematic diagram of the operating principle of an inertial navigation unit;
FIG. 2 is a schematic diagram of a temperature sensor formed by one single temperature sensing element;
FIG. 3 is a schematic diagram of a temperature sensor formed by a plurality of temperature sensing elements;
FIG. 4 is a schematic diagram of a temperature sensor with a planar array;
FIG. 5 is a schematic diagram of a temperature sensor with a curved array;
FIG. 6 is a schematic structural diagram of a device for guiding and releasing energy according to the present invention; and
FIG. 7 is a schematic diagram of a beauty instrument used as an embodiment of a device for guiding and releasing energy.

### DETAILED DESCRIPTION

The technical content of the present invention is further described in detail below with reference to the accompanying drawings and specific embodiments.

As described above, existing interventional energy therapy is still in an open-loop stage.

Therefore, the effect of energy releasing cannot be fed back in real time, and closed-loop control with the energy input cannot be implemented. It is taken into consideration that different lesion locations have different responses to the directionally released energy. For example, the foregoing laser rejuvenation cannot achieve an effect of real-time change. Therefore, after careful consideration, the inventor intends to use the three-dimensional skin temperature changes that occur when skin tissue receives interventional energy therapy as a basic condition of implementing closed-loop control on energy guiding and releasing. Three-dimensional skin temperature distribution data generated in this process is referred to as a three-dimensional skin temperature topographic map.

Therefore, the present invention first provides a method for generating a three-dimensional skin temperature topographic map. In this method, relative location of the inertial navigation unit and the temperature sensor remains unchanged. The inertial navigation unit is configured to record and draw a three-dimensional curve along which the temperature sensor moves on a skin surface, to obtain spatial location data in the three-dimension skin temperature topographic map. The temperature sensor is configured to record real-time temperature data at each spatial location simultaneously, to obtain temperature distribution data of skin tissue in a three-dimension space.

In the present invention, the inertial navigation unit obtains a corresponding location and a corresponding speed mainly by measuring the acceleration of an object with an accelerometer, and then performing mathematical operation such as integration. Additionally, an angular increment of an object is measured by using a gyroscope simultaneously, then being integrated to get an angle of an object, so as to correct the value of the acceleration of the object in an inertial system by the obtained angle of the object. Since the present invention is aim to be applied on skin tissue of a human body (particularly, facial skin or hand skin of a person), of which the space is relatively small and the spatial curvature is not smooth,the inertial navigation unit configured to implement the foregoing methods needs to have characteristics of high resolution and high sensitivity. In recent years, with rapid development of Micro Electro Mechanical Systems (MEMS) inertial device, the performance of the inertial navigation unit has already meeted navigation requirements and even surpasses. An inertial navigation system based on a multi-axis gyroscope and an accelerometer are widely applied to the fields of autonomous navigation for aviation, aerospace, sailing, and automobiles. Currently, the resolution and sensitivity of an MEMS inertial device (including a photoelectric displacement sensor) has already reached an order of magnitude at 10 ug and can satisfy a specific requirement of the foregoing use scenario.

It should be noted that although an existing MEMS inertial device has very high resolution and sensitivity, it is still difficult to apply the MEMS inertial device to the present invention without using an additional data processing measure. Specifically, an inertial device first has its error factor. For example, the gyroscope has a constant drift and a random walk, the accelerometer has a zero drift and a random error, and both the devices have different degrees of non-linear temperature drifts. Even if these factors are disregarded, the inertial navigation unit still cannot satisfy use requirements. In addition, the use scenario of the present invention further includes characteristics of a short operating distance and a low movement speed. This undoubtedly reduces energy of collected signals and also indicates that the inertial navigation unit of the present invention is in a working condition with a relatively low signal-to-noise ratio. Therefore, the inertial navigation unit needs to have a higher data processing capability.

In view of the foregoing cases, two data processing functions are additionally added to the inertial navigation unit of the present invention: 1. super-resolution processing; and 2. a deep learning network.

The super-resolution processing is a method restoring a high-resolution image from a low-resolution image or an image sequence. The image super-resolution technology includes super-resolution restoration and super-resolution reconstruction. A common operation of the image super-resolution technology is interpolation. Specifically, interpolation processing is performed on data of a low sampling rate to generate data with a high sampling rate and to ensure an adequate data recovery effect. In the present invention, an objective of applying the super-resolution processing to the inertial navigation unit is to restore the original navigation data of a relatively low signal-to-noise ratio and a relatively low sampling rate to data of high sampling rate and relatively high quality for subsequent use.

The deep learning is combining low level features into abstract high-level features which represents of attribute categories or attributes features, to discover distributed feature representation of data. The purpose is to establish a neural network that simulates a human brain for analysis and learning, and the neural network simulates the mechanism of a human brain to interpret data such as images, sound, and text. In the present invention, according to the used samples of actual working conditions, a better super-resolution processing manner trained by using the deep learning network is used, thereby further improving navigation precision.

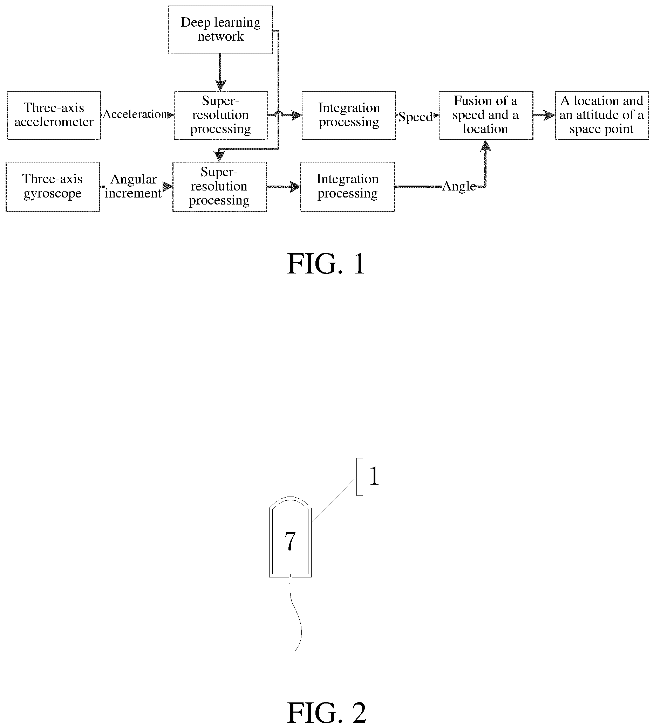

As shown in FIG. 1, the inertial navigation unit of the present invention at least includes a three-axis accelerometer and a three-axis gyroscope. The three-axis accelerometer is configured to collect acceleration, and the three-axis gyroscope is configured to collect angular increments. During collection, super-resolution processing needs to be performed to improve sampling precision. In addition, the super-resolution processing manner is continuously optimized along with the use of the deep learning network, to further optimize a data collection result. A speed parameter is obtained by performing integration processing on the data collected by the three-axis accelerometer. An angle parameter is obtained by performing integration processing on the data collected by the three-axis gyroscope. The speed parameter and the angle parameter are combined with each other, so that the speed and the location may be conbined, to further obtain the spatial location data.

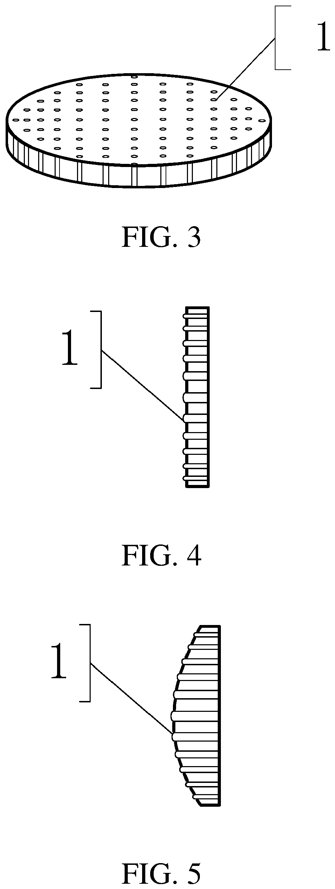

FIG. 2 to FIG. 5 respectively show different examples of a temperature sensor used in the present invention. FIG. 2 shows a temperature sensor formed by one single temperature sensing element, FIG. 3 is a schematic top view of a temperature sensor formed by a plurality of temperature sensing elements, FIG. 4 is a schematic side view of a temperature sensor formed by a plurality of temperature sensing elements distributed in a planar array, and FIG. 5 is a schematic side view of a temperature sensor formed by a plurality of temperature sensing elements distributed in a curved array.

In the embodiments of the present invention, a temperature sensing element 7 may be, but is not limited to, a thermal couple, and may be a contact sensing element such as a temperature sensitive diode or a platinum thin-film thermistor on a polyimide substrate, or a non-contact sensing element such as an infrared temperature measurement sensor, an infrared array temperature measurement sensor, or an imaging CCD temperature measurement sensor.

A temperature sensor 1 may be formed by one single temperature sensing element 7 shown in FIG. 2, or may be formed by the plurality of temperature sensing elements 7 distributed in a planar array shown in FIG. 4, or may be formed by the plurality of temperature sensing elements 7 distributed in a curved array shown in FIG. 5. However, the temperature sensor is not limited to these structural forms. An objective of using the plurality of temperature sensing elements 7 is for design redundancy or using a fused result of sensed temperature values of different sensing elements to sense the temperature of skin tissue more accurately. During actual application, the temperature sensing element 7 may be designed into different sizes according to different application conditions, and the distances between these sensing elements may be adjusted as required, making the temperature sensing element 7 applicable to the measurement of three-dimensional skin temperature for different skin locations and different area sizes.

As discussed above, relative locations of the inertial navigation unit and the temperature sensor remains unchanged. Therefore, spatial location data of the temperature sensor may be obtained by using the spatial location data obtained by the inertial navigation unit. Temperature distribution data of skin tissue in a three-dimensional space may be obtained by combining the real-time temperature data at each spatial location collected in real time by the temperature sensor, to further draw a three-dimensional skin temperature topographic map.

Based on the three-dimensional skin temperature topographic map, the present invention provides a method for guiding and releasing energy based on a three-dimensional skin temperature topographic map. In the method for guiding and releasing energy, on one hand, interventional energy therapy is performed on skin tissue by using a manner of releasing energy such as radio frequency energy, ultrasonic energy, laser energy, or microwave energy, and dynamic response temperatures of the skin tissue may be measured by using the temperature sensors. The dynamic response temperatures of the skin tissue are fed back through closed-loop control, to dynamically adjust a release speed and a release direction of interventional energy, so that the real-time temperature of the skin tissue is controlled to be within an optimal temperature range of the interventional energy therapy. On the other hand, a real-time three-dimensional skin temperature topographic map may be obtained by combining the inertial navigation unit and the temperature sensor. The three-dimensional skin temperature topographic map is displayed by using a display apparatus (for example, a personal computer (PC) monitor, a tablet computer, or the like), an operator knows an external effect (specifically represented as the dynamic response temperatures of the skin tissue) of the interventional energy therapy in real time and adjusts the release speed and the release direction of the interventional energy correspondingly.

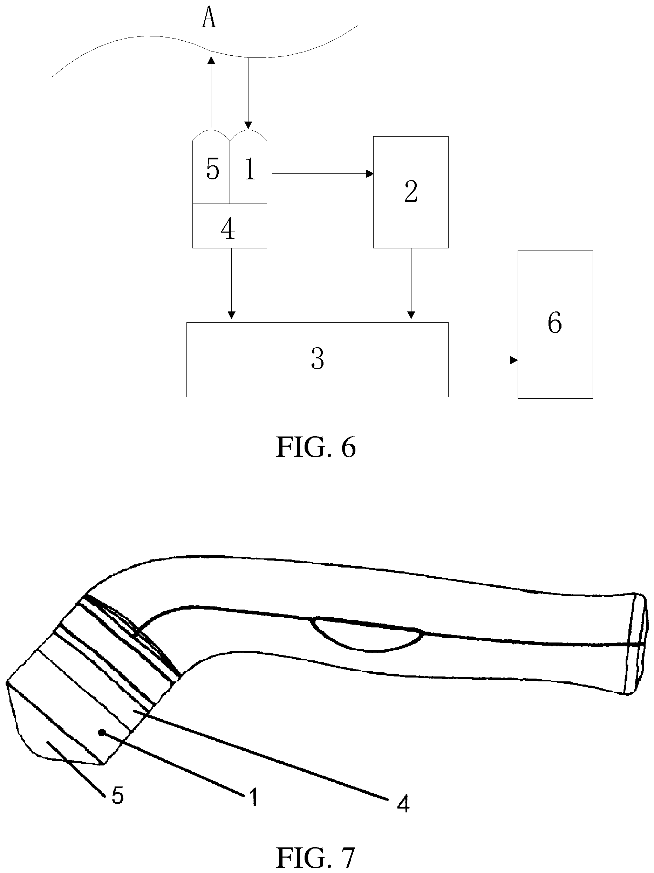

Furthermore, the present invention further provides a device for guiding and releasing energy based on a three-dimensional skin temperature topographic map. As shown in FIG. 6, the device includes a temperature sensor 1, a data collection apparatus 2, a microprocessor 3, an inertial navigation unit 4, an interventional energy release unit 5, and a display apparatus 6. The temperature sensor 1, the interventional energy release unit 5, and the inertial navigation unit 4 are fixed together and are tightly attached to the surface (an area labelled A in FIG. 6) of skin tissue during use. Temperature data collected by the temperature sensor 1 is input into the data collection apparatus 2 and then enters the microprocessor 3 after analogy-to-digital conversion. In addition, spatial location data collected by the inertial navigation unit 4 also enters the microprocessor 3, for being then processed by the microprocessor 3. A processing result of the microprocessor 3 may be input into the display apparatus 6 for display. For example, the three-dimensional skin temperature topographic map is displayed in real time on the display apparatus 6. In addition, a storage unit (for example, a non-volatile memory such as a flash and an electrically-erasable programmable read-only memory (E²PROM)) may be disposed in the device for guiding and releasing energy as required, and the storage unit is connected to the microprocessor 3 and the display apparatus 6 (not shown in the figure).

An obvious characteristic of the device for guiding and releasing energy is that the temperature sensor 1 and the interventional energy release unit 5 are dynamically closed-loop controlled. The interventional energy of the interventional energy release unit 5 is dynamically adjusts and controlled with a closed-loop control algorithm, according to the temperature data collected in real time by the temperature sensor 1, so that the temperature of human body skin enters an optimal temperature range of the interventional energy therapy as soon as possible. Specifically, each of the temperature sensors 1 collects an accurate temperature value of the skin tissue at which the temperature sensor is located, compares this accurate temperature value with a temperature value within the optimal temperature range that is clinically verified, and sends a comparison result into the microprocessor 3; and the closed-loop control algorithm is executed in the microprocessor 3 to obtain a control value for the interventional energy release unit 5 so as to accurately control parameters such as an energy value and an energy frequency of the energy released by the interventional energy release unit 5, thereby eventually controlling the temperature value of the skin tissue to be within the optimal temperature range and so achieving a dynamic balance between interventional energy therapy and dynamic monitoring and diagnosis.

In addition, the microprocessor 3 is further responsible for receiving inertial information such as a speed, a location, and an attitude output by the inertial navigation unit 4, and calculating, according to the inertial information, the three-dimensional locations along which the temperature sensor 1 or the interventional energy release unit 5 passes. And then the microprocessor 3 outputs a three-dimensional curve with skin temperature data to the display apparatus 6 in real time by combining the accurate skin temperature monitored by the temperature sensor 1 with the three-dimensional locations. The display apparatus 6 displays the three-dimensional skin temperature topographic map according to the three-dimensional locations and the real-time skin temperature . The skin temperature data beyond the optimal temperature range may be displayed with an alert in a manner of sound, an image, or a combination thereof, so that an operator is provided with intuitive and clear operation instructions.

In an embodiment of the present invention, the device for guiding and releasing energy is a beauty instrument implementing a skin care function. As shown in FIG. 7, the head of the beauty instrument is the interventional energy release unit 5. In other embodiments of the present invention, the interventional energy release unit 5 may be any one of an ultrasonic generator, a radio frequency transmitter, or a pulse laser. The temperature sensor 1 is closely attached near the interventional energy release unit 5. It should be noted that the temperature sensor 1 may be implemented in various forms such as the foregoing thermal couple, temperature sensitive diode, or platinum thin-film thermistor. Therefore, a specific mounting location of the temperature sensor 1 does not need to be fixed as long as the real-time temperature data of the skin tissue can be adequately collected. The inertial navigation unit 4 may be disposed at a rear end of the temperature sensor 1. The inertial navigation unit 4 includes a three-axis accelerometer and a three-axis gyroscope. Location relationships of the three-axis accelerometer and the three-axis gyroscope relative to the temperature sensor 1 are fixed. Therefore, the spatial location data of the temperature sensor 1 may be obtained according to the spatial location data collected by the inertial navigation unit 4. In different embodiments of the present invention, the data collection apparatus 2 may be an analog-to-digital/digital-to-analog conversion unit, or may be another signal conversion unit or data communications unit. The microprocessor 3 may be any one of a central processing unit (CPU), a microcontroller unit (MCU), or a single chip microcomputer.

In the embodiment shown in FIG. 7, the data collection apparatus 2 and the microprocessor 3 may be embedded at middle or rear of the beauty instrument. The display apparatus may be a PC monitor, a tablet computer, or a smartphone. The beauty instrument and the display apparatus are connected in a wired/wireless manner. In other embodiments, the microprocessor and the display apparatus may be implemented by using a corresponding functional component in a PC, a tablet computer, or a smartphone. The data collection apparatus 2 is built in the beauty instrument and exchanges data with an external microprocessor in a wired/wireless manner.

Compared with the prior art, the device for guiding and releasing energy provided in the present invention performs dynamic closed-loop control on the temperature sensor and the interventional energy release unit, and a temperature value of skin tissue is eventually controlled to be within an optimal temperature range to achieve dynamic balance between interventional energy therapy and dynamic monitoring and diagnosis. The three-dimensional skin temperature topographic map is displayed according to the spatial location data and the real-time temperature data, so that an operator is provided with intuitive and clear operation instructions.

The method and device for guiding and releasing energy based on a three-dimensional skin temperature topographic map provided in the present invention are described in detail above. For a person of ordinary skill in the art, any obvious modifications made to the present invention will be included in the present invention.

## Claims

1. A method for generating a three-dimensional skin temperature topographic map, for being applied in a device including an inertial navigation unit and a temperature sensor whose relative location remains unchanged, comprises the following steps:
the inertial navigation unit recording a three-dimensional curve along which the temperature sensor moves on a skin surface, to obtain spatial locations data, and the temperature sensor recording real-time temperature data at each of the spatial locations simultaneously; and
integrating the spatial locations data and corresponding real-time temperature data to obtain a three-dimensional skin temperature topographic map.

2. The method for generating a three-dimensional skin temperature topographic map according to claim 1, further comprising:
obtaining, by the inertial navigation unit, an acceleration a location and a speed of the device by performing integration on the acceleration of measured by an accelerometer; and obtaining an angle of an object by integrating an angular increment of an object measured by a gyroscope, and correcting the acceleration of the object in an inertial system by using angle information.

3. A method for guiding and releasing energy based on a three-dimensional skin temperature topographic map, comprising the following steps:
performing interventional energy therapy on skin tissue, and measuring a dynamic response temperature of the skin tissue by using a temperature sensor; and
feeding back the dynamic response temperature of the skin tissue through closed-loop control, to dynamically adjust a release speed and a release direction of interventional energy.

4. The method for guiding and releasing energy according to claim 3, further comprising the following steps:
generating a real-time three-dimensional skin temperature topographic map by combining the inertial navigation unit and the temperature sensor; and
displaying the three-dimensional skin temperature topographic map by using a display apparatus, so that an operator knows an external effect of the interventional energy therapy in real time and adjusts the release speed and release direction of interventional energy in a targeted manner.

5. A device for guiding and releasing energy based on a three-dimensional skin temperature topographic map, comprising a temperature sensor, a data collection apparatus, a microprocessor, an inertial navigation unit, and an interventional energy release unit, wherein
the temperature sensor, the interventional energy release unit, and the inertial navigation unit are fixed together and are tightly attached to skin tissue during use;
real-time temperature data collected by the temperature sensor is input into the data collection apparatus to enter the microprocessor; and
the microprocessor dynamically adjusts and controls interventional energy of the interventional energy release unit according to the real-time temperature data collected by the temperature sensor.

6. The device for guiding and releasing energy according to claim 5, wherein
spatial location data collected by the inertial navigation unit also enters the microprocessor; and the microprocessor integrates the spatial location data and the corresponding real-time temperature data to obtain a three-dimensional skin temperature topographic map and displays the three-dimensional skin temperature topographic map in real time by using a display apparatus.

7. The device for guiding and releasing energy according to claim 5, wherein
the temperature sensor collects in real time a temperature value of the skin tissue at which the temperature sensor is located, compares this accurate temperature value with a temperature value within an optimal temperature range that is clinically verified, and sends a comparison result into the microprocessor; and a closed-loop control algorithm is performed in the microprocessor to control a value and a frequency of energy released by the interventional energy release unit, to control the temperature value of the skin tissue to be within the optimal temperature range.

8. The device for guiding and releasing energy according to any one of claims 5 to 7, wherein
the device for guiding and releasing energy is a beauty instrument implementing a skin care function.

9. The device for guiding and releasing energy according to claim 8, wherein
the interventional energy release unit is any one of an ultrasonic generator, a radio frequency transmitter, or a pulse laser.

10. The device for guiding and releasing energy according to any one of claims 5 to 7, wherein
the temperature sensor is a thermal couple, a temperature sensitive diode, a platinum thin-film thermistor, or any one of an infrared temperature measurement sensor, an infrared array temperature measurement sensor, or an imaging charge-coupled device (CCD) temperature measurement sensor.
